# EUROPÄISCHE PATENTSCHRIFT

(11) **EP 0 577 637 B1**
(45) Veröffentlichungstag und Bekanntmachung des Hinweises auf die Patenterteilung: **04.09.1996**
(21) Anmeldenummer: 92906620.7
(22) Anmeldetag: 18.03.1992
(51) Int. Cl.: C07C 41/03, C07C 43/11

(54) **VERBESSERTES VERFAHREN ZUR HERSTELLUNG VON NICHTIONISCHEN OBERFLÄCHENAKTIVEN VERBINDUNGEN**
IMPROVED PROCESS FOR PRODUCING NON-IONIC SURFACE-ACTIVE COMPOUNDS
PROCEDE AMELIORE DE PREPARATION DE COMPOSES TENSIO-ACTIFS NON IONIQUES

(30) Priorität: 27.03.1991 DE 4110178
(43) Veröffentlichungstag der Anmeldung: 12.01.1994
(73) Patentinhaber: Henkel Kommanditgesellschaft auf Aktien, 40191 Düsseldorf (DE)
(72) Erfinder: BEHLER, Ansgar, D-4250 Bottrop (DE); ENDRES, Helmut, D-4018 Langenfeld (DE); RATHS, H.-Christian, D-4019 Monheim (DE); PLOOG, Uwe, D-5657 Haan (DE)
(86) Internationale Anmeldenummer: EP9200587
(87) Internationale Veröffentlichungsnummer: WO9217429

(56) Entgegenhaltungen:
- EP-A- 0 398 450
- GB-A- 949 891
- US-A- 4 721 817
- US-A- 4 727 199

## Beschreibung

Die Erfindung betrifft ein verbessertes Verfahren zur Herstellung von nichtionischen oberflächenaktiven Verbindungen durch Alkoxylierung von Verbindungen mit aktiven Wasserstoffatomen in Gegenwart schwerlöslicher getemperter Erdalkaliphosphate.

Anlagerungsprodukte von Ethylen- und/oder Propylenoxid an primäre Alkohole, sogenannte "Alkoholalkoxylate", besitzen als nichtionihe Tenside infolge ihrer ausgezeichneten Detergenseigenschaften und ihrer hohen Kaltwasserlöslichkeit große Bedeutung für die Herstellung von Wasch-, Spül- und Reinigungsmitteln. Im Verlauf der Alkoxylierung, die in der Regel in Gegenwart von leicht löslichen Alkalihydroxiden oder -alkoholaten durchgeführt wird, kommt es jedoch nicht zu einer selektiven Anlagerung einer diskreten Anzahl von Ethylen- und/oder Propylenoxideinheiten an jeweils ein Molekül des Alkohols, die Reaktion folgt vielmehr statistischen Gesetzen und führt zu einem Gemisch homologer Additionsprodukte, deren Alkoxylierungsgrade ein breites Spektrum umfassen.

Aus **J.Am.Oil.Chem.Soc. 63, 691 (1986)** und **HAPPI 52 (1986)** ist bekannt, daß die Verteilung der Alkoxylierungsgrade im Gemisch der Alkoholalkoxylate, die sogenannte "Homologenverteilung", die Eigenschaften der erhaltenen Additionsprodukte maßgeblich beeinflußt. Dabei wurde gefunden, daß Produkte mit "eingeengter" Homologenverteilung, sogenannte "narrow-range alkoxylates", Vorteile gegenüber vergleichbaren Produkten mit "breiter" Homologenverteilung aufweisen, so z. B.:
- niedrigere Fließpunkte,
- höhere Rauchpunkte,
- geringere Anzahl von Molen Alkylenoxid zum Erreichen der Wasserlöslichkeit,
- geringere Anteile an nichtumgesetzten Alkohol und damit verbunden, eine verminderte Geruchsbelastung sowie
- Reduzierung des Plumings beim Sprühtrocknen von Alkoholalkoxylat-enthaltenden Waschmittelslurries.

In der Vergangenheit hat es nicht an Versuchen gemangelt, durch Variation des Alkoxylierungskatalysators, Anlagerungsprodukte von Ethylen- und/oder Propylenoxid an primäre Alkohole herzustellen, die eine eingeengte Homologenverteilung aufweisen und über das geschilderte Eigenschaftsprofil verfügen.

So sind aus den Europäischen Patentanmeldungen **EP 0 006 105 A1** und **EP 0 033 760 A1** Verfahren bekannt, die dieses Ziel unter Verwendung von Erdalkalimetalloxiden und -hydroxiden erreichen. Gemäß den Lehren der Europäischen Patentschriften **EP 0 092 256 B1** und **EP 0 115 084 B1** lassen sich auch Erdalkalimetallalkoxide und -phenoxide in Kombination mit Phosphorsäure sowie gemäß der US-Patentschrift **US 4,721,817** Aluminiumalkoholate bzw. -phenolate in Verbindung mit phosphorhaltigen Säuren als Katalysatoren zur Herstellung von Alkoxylierungsprodukten mit eingeengter Homologenverteilung verwenden. Alle genannten Katalysatorsysteme sind jedoch homogen, d. h. im Reaktionsgemisch vollständig löslich und können daher die anwendungstechnischen Eigenschaften der Alkoxylierungsprodukte negativ beeinflussen, da ihre Abtrennung aus dem Reaktionsgemisch technisch aufwendig ist.

Man hat versucht, dieses Problem durch Verwendung heterogener, im Reaktionsgemisch schwerlöslicher Katalysatorsysteme, wie z. B. Hydrotalcit [**DE 38 43 713 A1**], Magnesium-/Aluminiumsalze [**DE 38 33 076 A1**], Zirkoniumoxysulfat [**US 4,727,199**] oder Kombinationen aus Calciumsalzen, Aluminiumtrialkoxiden und anorganischen Säuren [**US 4,775,653**] zu lösen. In der EP 0 398 450 A2 ist die Verwendung von Bariumphosphat, welches zum Zweck der Trocknung (Wasserentzug) erhitzt wurde, beschrieben. Die Trocknungstemperatur wird in diesem Zusammenhang als unkritisch bezeichnet, sofern sie nicht 600°C überschreitet, so daß es zur Bildung von Pyrophosphat käme. Die genannten Systeme bereiten infolge ihrer Feinteiligkeit Probleme bei der Abtrennung, lassen sich nur mit hohem technischen Aufwand handhaben, weisen keine ausreichende Aktivität auf oder führen zu Alkoxylierungsprodukten, deren Homologenverteilung keine zufriedenstellende Einengung erkennen läßt.

Der Erfindung lag somit die Aufgabe zugrunde, ein Verfahren zur Herstellung von nichtionischen oberflächenaktiven Verbindungen zu entwickeln, das frei von den geschilderten Nachteilen ist.

Gegenstand der Erfindung ist ein verbessertes Verfahren zur Herstellung von nichtionischen oberflächenaktiven Verbindungen durch Alkoxylierung von Verbindungen mit aktiven Wasserstoffatomen in Gegenwart von im Reaktionsgemisch schwerlöslichen Erdalkalisalzen, bei dem man als Alkoxylierungskatalysatoren bei 180 bis 300°C getemperte Erdalkaliphosphate verwendet und diese nach Beendigung der Alkoxylierung durch Filtration abtrennt.

Überraschenderweise wurde gefunden, daß getemperte Erdalkaliphosphate gegenüber anderen Erdalkalimetallsalzen, insbesondere auch nichtgetempertem Strontium- oder Bariumphosphat, eine höhere Aktivität als Alkoxylierungskatalysator besitzt, was sich in kürzeren Reaktionszeiten und einer schärferen Einengung der Homologenverteilung dokumentiert. Das Verfahren weist ferner den Vorteil auf, daß sich die getemperten Erdalkaliphosphate leicht abtrennen und - gegebenenfalls nach Aufarbeitung - in die Alkoxylierung zurückführen lassen.

Unter Verbindungen mit aktiven Wasserstoffatomen im Sinne der Erfindung sind z. B. Fettsäuren, Hydroxyfettsäuren, Alkylphenole, Polyglycole, Fettamine, Fettsäurealkanolamide, vicinale hydroxy, alkoxy-substituierte Alkane, sekundäre Alkohole, insbesondere aber Fettalkohole zu verstehen.

In einer speziellen Ausführungsform der Erfindung werden nicht-ionische oberflächenaktive Verbindungen der Formel (I) hergestellt, 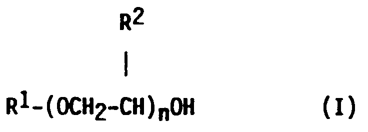
in der R¹ für lineare oder verzweigte aliphatische oder cycloaliphatische Kohlenwasserstoffreste mit 6 bis 44 Kohlenstoffatomen und 0, 1, 2 oder 3 Doppelbindungen, R² für Wasserstoff oder eine Methylgruppe und n für ganze oder gebrochene Zahlen von 1 bis 20 stehen.

Dabei geht man von primären Alkoholen der Formel **(II)** aus, 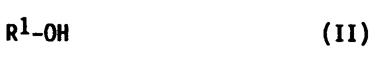
in der R¹ die oben angegebene Bedeutung hat. Als typische Beispiele für primäre Alkohole mit linearem Kohlenwasserstoffrest kommen Capronalkohol, Caprylalkohol, Caprinalkohol, Cetylalkohol, Stearylalkohol, Oleylalkohol, Elaidylalkohol, Petroselinylalkohol, Linolylalkohol, Linolenylalkohol, Behenylalkohol oder Erucylalkohol in Betracht. Nichtionische oberflächenaktive Verbindungen mit besonders vorteilhaften Detergenseigenschaften werden erhalten, wenn man von linearen, gesättigten primären Alkoholen ausgeht, die 12 bis 14 Kohlenstoffatome enthalten. Aus diesem Grund ist die Verwendung von Myristyl- und Laurylalkohol bevorzugt.

Wie in der Fettchemie üblich, können diese Alkohole auch in Form technischer Gemische vorliegen, wie sie z. B. durch Hochdruckhydrierung von Fettsäuremethylesterschnitten pflanzlicher oder tierischer Herkunft oder durch Hydrierung von technischen Aldehydfraktionen aus der Roelen'schen Oxosynthese zugänglich sind.

Als typische Beispiele für primäre Alkohole mit verzweigtem Kohlenwasserstoffrest kommen Guerbetalkohole in Betracht, die durch Oxidation primärer Alkohole zu den entsprechenden Aldehyden, nachfolgende Aldolkondensation, Dehydrierung des entstandenen Aldols und abschließende Hydrierung des gebildeten Allylaldehyds zugänglich sind [**Soap.Cosm.Chem.Spec., 52 (1987)**]. Nichtionische oberflächenaktive Verbindungen mit besonders vorteilhaften Detergenseigenschaften werden erhalten, wenn man von Guerbetalkoholen mit 16 bis 36 Kohlenstoffatomen ausgeht. Aus diesem Grund ist die Verwendung von 2-Octyl-dodecanol-1 bevorzugt.

Unter getemperten Erdalkaliphosphaten sind die Magnesium-, Calcium-, Strontium- und Bariumsalze der Phosphorsäure sowie deren Gemische zu verstehen, die man z. B. durch Erhitzen von kristallwasserhaltigen Erdalkalimetallhydroxiden mit 85 gew.-%iger Phosphorsäure und anschließender Wärmebehandlung (tempern) der resultierenden Phosphate bei 180 bis 300, vorzugsweise 220 bis 280°C erhält. Da getempertes Strontiumphosphat in der Alkoxylierung eine besonders deutliche Einengung der Homologenverteilung bewirkt, toxikologisch unbedenklich ist und im Anschluß an die Alkoxylierung durch Filtration problemlos abgetrennt werden kann, ist seine Verwendung bevorzugt.

Die Alkoxylierung von Verbindungen mit aktiven Wasserstoffatomen kann in Gegenwart von 0,1 bis 5, vorzugsweise 0,4 bis 2 Gew.-% - bezogen auf das Endprodukt der Alkoxylierung - eines getemperten Erdalkaliphosphats durchgeführt werden.

Die Alkoxylierung kann bei Temperaturen von 120 bis 220°C und Drücken von 1 bis 5 bar durchgeführt. Im Hinblick auf eine ausreichende Reaktionsgeschwindigkeit einerseits und einer möglichst geringen thermischen Belastung der Produkte andererseits, hat es sich als optimal erwiesen, die Alkoxylierung bei Temperaturen von 160 bis 190°C durchzuführen.

Die Alkoxylierung kann mit Ethylen- oder Propylenoxid sowie mit Gemischen beider (in Random- oder Blockverteilung) durchgeführt werden, wobei ein Mol des primären Alkohols mit durchschnittlich 1 bis 20 Mol des Alkylenoxids umgesetzt wird. Da die Alkoxylierung einem statistischen Verlauf unterliegt, gibt die Indexzahl n in Formel **(I)** den durchschnittlichen Alkoxylierungsgrad an und kann somit ganzzahlige oder gebrochene Werte - auch kleiner 1 - annehmen.

Die folgenden Beispiele sollen den Gegenstand der Erfindung näher erläutern, ohne ihn darauf einzuschränken, wobei auf Abbildungen Bezug genommen wird. Die Abbildungen zeigen die gemäß den Beispielen beziehungsweise Vergleichsbeispielen erzielten Homologenverteilungen.

### Beispiele

### I. Herstellung der Katalysatoren:

**Strontiumphosphat, getempert (A).** 239 g (0,9 mol) Strontiumhydroxidoctahydrat wurden in 300 ml Wasser über einen Zeitraum von 40 min auf 90°C erhitzt und portionsweise mit 73 g (0,6 mol) 85 gew.-%iger Phosphorsäure versetzt. Nach Beendigung der Zugabe ließ man 15 min nachreagieren, anschließend wurde das Wasser mit Hilfe eines Rotationsverdampfers abgetrennt. Die vollständige Entwässerung des Produktes erfolgte danach in einem Vakuumtrockenschrank bei 120°C. Der pH-Wert einer 1 gew.-%igen Aufschlämmung in Wasser betrug 12,2.

Anschließend wurde das Strontiumphosphat über einen Zeitraum von 2 h bei 260°C getempert. Der pH-Wert einer 1 gew.-%igen Aufschlämmung betrug 11,9.

**Strontiumphosphat (B).** Die Herstellung von A wurde wiederholt, auf eine Temperung jedoch verzichtet.

### II. Alkoxylierungen:

### Beispiel 1:

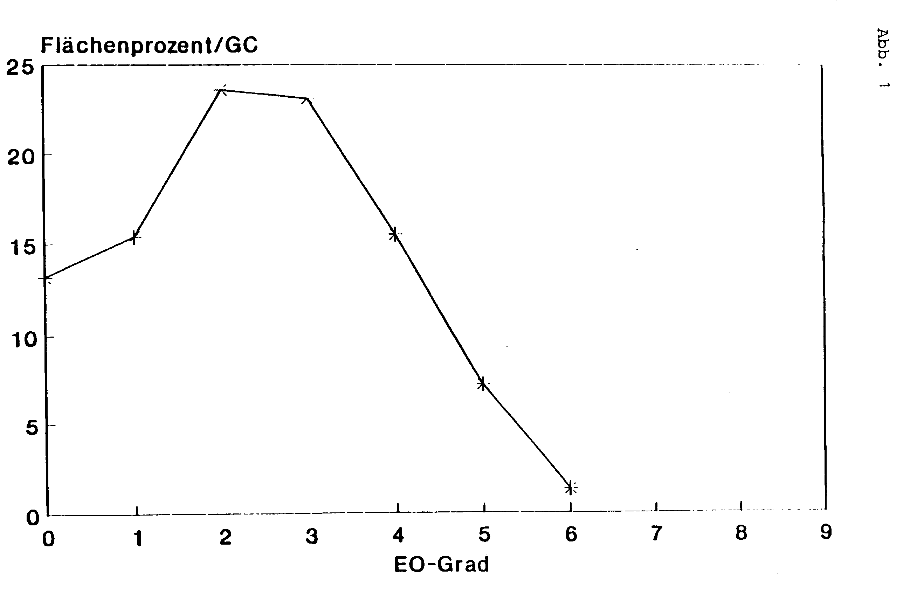

**Dodecanol + 2 Mol Ethylenoxid.** 6 g des Katalysators A wurden in 407 g (2,2 mol) Dodecanol (Lorol^{(R)} C12, Hydroxylzahl 298, Fa. Henkel KGaA) gelöst und in einen Autoklaven überführt. Es wurde mit Stickstoff gespült und 30 min bei 100°C evakuiert. Im Anschluß wurden bei 180°C und einem Druck von max. 5 bar 193 g (4,4 mol) Ethylenoxid innerhalb von 90 min portionsweise aufgedrückt. Nach Beendigung der Ethylenoxidzugabe ließ man 30 min nachreagieren. Der Katalysator wurde über eine Nutsche abfiltriert. Die Ausbeute betrug 601 g. Die Homologenverteilung ist Abb.1 zu entnehmen.

### Vergleichsbeispiel 1:

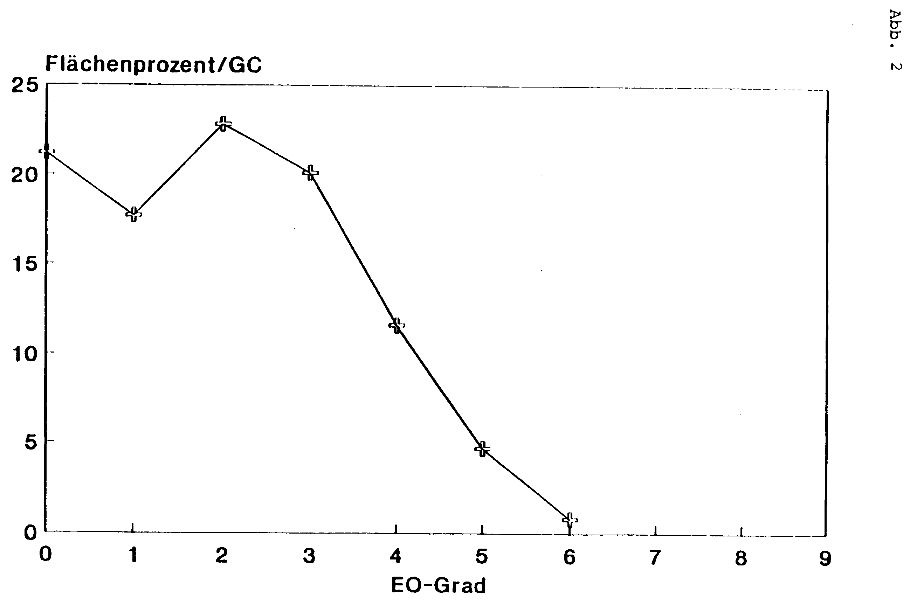

**Dodecanol + 2 mol Ethylenoxid.** Beispiel 1 wurde unter Verwendung von 6 g des Katalysators B wiederholt. Nach einer Reaktionszeit von 120 min wurden 600 g eines Ethoxylierungsproduktes erhalten. Die Homologenverteilung ist Abb.2 zu entnehmen.

### Beispiel 2:

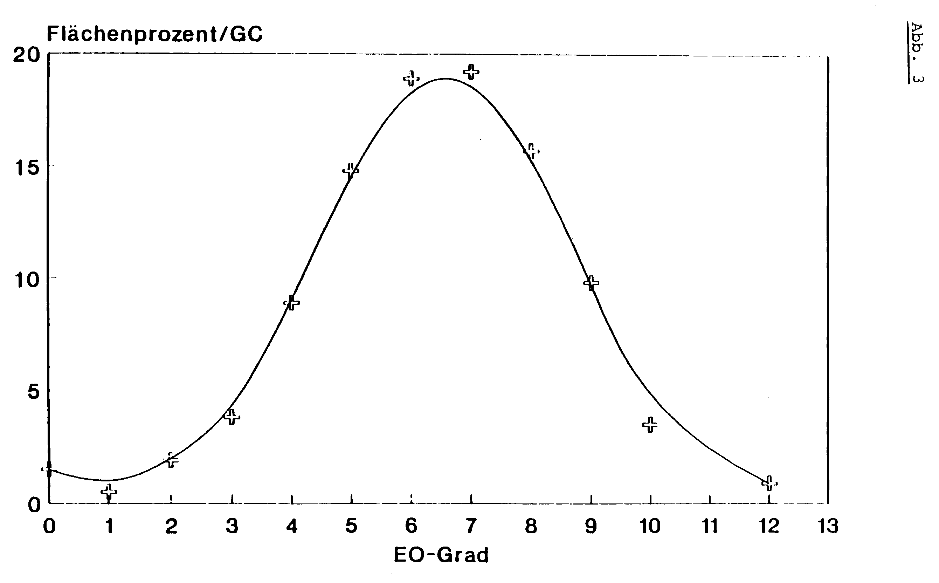

**Dodecanol + 6,5 mol Ethylenoxid.** Beispiel 1 wurde unter Verwendung von 6 g des Katalysators A, 407 g Dodecanol und 630 g (14,3 mol) Ethylenoxid wiederholt. Nach einer Reaktionszeit von 240 min wurden 1031 g eines Ethoxylierungsproduktes erhalten, das eine Hydroxylzahl von 115,6 aufwies. Die Homologenverteilung ist Abb.3 zu entnehmen.

### Vergleichsbeispiel 2:

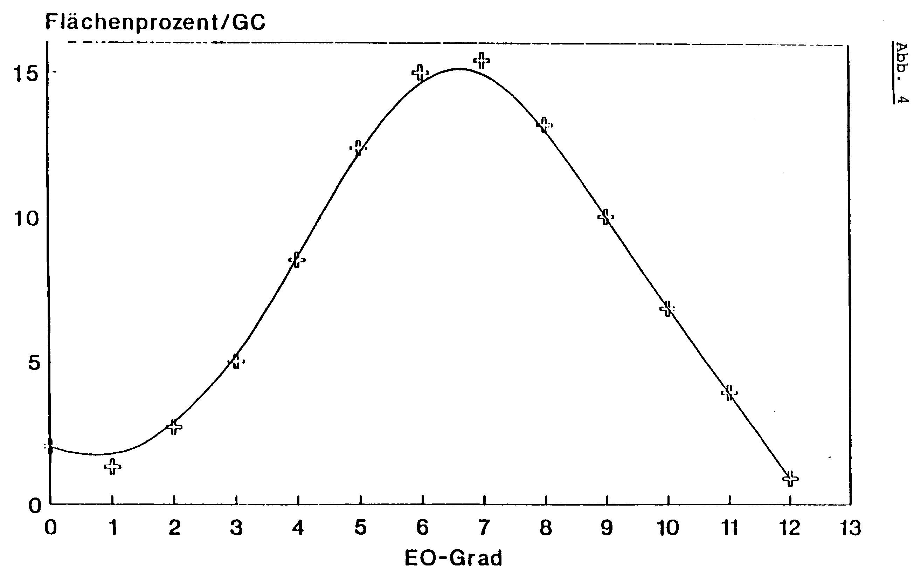

**Dodecanol + 6,5 mol Ethylenoxid.** Beispiel 2 wurde unter Verwendung von 6 g des Katalysators B wiederholt. Nach einer Reaktionszeit von 270 min wurden 1030 g eines Ethoxylierungsproduktes erhalten, das eine Hydroxylzahl von 118,4 aufwies. Die Homologenverteilung ist Abb.4 zu entnehmen.

## Patentansprüche

1. Verbessertes Verfahren zur Herstellung von nichtionischen oberflächenaktiven Verbindungen durch Alkoxylierung von Verbindungen mit aktiven Wasserstoffatomen in Gegenwart von im Reaktionsgemisch schwerlöslichen Erdalkalisalzen, **dadurch gekennzeichnet**, daß man als Alkoxylierungskatalysatoren bei 180 bis 300°C getemperte Erdalkaliphosphate verwendet und diese nach Beendigung der Alkoxylierung durch Filtration abtrennt.

2. Verfahren nach Anspruch 1, **dadurch gekennzeichnet**, daß die Verbindungen mit aktiven Wasserstoffatomen primäre Alkohole darstellen.

3. Verfahren nach mindestens einem der Ansprüche 1 und 2, **dadurch gekennzeichnet**, daß man die Alkoxylierung mit Ethylenoxid und/oder Propylenoxid durchführt.

4. Verfahren nach mindestens einem der Ansprüche 1 bis 3, **dadurch gekennzeichnet**, daß man als Alkoxylierungskatalysator getempertes Strontiumphosphat verwendet.

5. Verfahren nach mindestens einem der Ansprüche 1 bis 4, **dadurch gekennzeichnet**, daß die nichtionischen oberflächenaktiven Verbindungen der Formel **(I)** folgen, 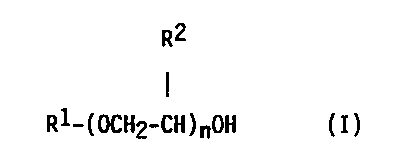 in der R¹ für einen linearen oder verzweigten aliphatischen oder cycloaliphatischen Kohlenwasserstoffrest mit 6 bis 44 Kohlenstoffatomen und 0, 1, 2 oder 3 Doppelbindungen, R² für Wasserstoff oder eine Methylgruppe und n für ganze oder gebrochene Zahlen von 1 bis 20 stehen.

6. Verfahren nach mindestens einem der Ansprüche 1 bis 5, **dadurch gekennzeichnet**, daß R¹ für einen linearen, gesättigten, aliphatischen Kohlenwasserstoffrest mit 12 bis 14 Kohlenstoffatomen steht.

7. Verfahren nach mindestens einem der Ansprüche 1 bis 5, **dadurch gekennzeichnet**, daß R¹ für einen verzweigten, gesättigten aliphatischen Kohlenwasserstoffrest mit 16 bis 36 Kohlenstoffatomen steht.

8. Verfahren nach mindestens einem der Ansprüche 1 bis 7, **dadurch gekennzeichnet**, daß man die Alkoxylierung bei Temperaturen von 120 bis 220°C und Drücken von 1 bis 5 bar durchführt.

9. Verfahren nach mindestens einem der Ansprüche 1 bis 8, **dadurch gekennzeichnet**, daß man die Alkoxylierung in Gegenwart von 0,1 bis 5,0 Gew.-%, bezogen auf das Endprodukt der Alkoxylierung, des Erdalkaliphosphates durchführt.

10. Verfahren nach mindestens einem der Ansprüche 1 bis 9, **dadurch gekennzeichnet**, daß man die Temperung der Erdalkaliphosphate bei Temperaturen von 180 bis 300°C durchführt.

## Claims

1. An improved process for the production of nonionic surface-active compounds by alkoxylation of compounds containing active hydrogen atoms in the presence of alkaline earth metal salts sparingly soluble in the reaction mixture, characterized in that alkaline earth metal phosphates heated at 180 to 300°C are used as the alkoxylation catalysts and are removed by filtration on completion of the alkoxylation reaction.

2. A process as claimed in claim 1, characterized in that the compounds containing active hydrogen atoms are primary alcohols.

3. A process as claimed in at least one of claims 1 and 2, characterized in that the alkoxylation is carried out with ethylene oxide and/or propylene oxide.

4. A process as claimed in at least one of claims 1 to 3, characterized in that heated strontium phosphate is used as the alkoxylation catalyst.

5. A process as claimed in at least one of claims 1 to 4, characterized in that the nonionic surface-active compounds correspond to formula (I): 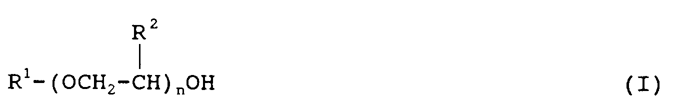 in which R¹ is a linear or branched aliphatic or cycloaliphatic hydrocarbon radical containing 6 to 44 carbon atoms and 0, 1, 2 or 3 double bonds, R² is hydrogen or a methyl group and n stands for whole or broken numbers of 1 to 20.

6. A process as claimed in at least one of claims 1 to 5, characterized in that R¹ is a linear, saturated aliphatic hydrocarbon radical containing 12 to 14 carbon atoms.

7. A process as claimed in at least one of claims 1 to 5, characterized in that R¹ is a branched, saturated aliphatic hydrocarbon radical containing 16 to 36 carbon atoms.

8. A process as claimed in at least one of claims 1 to 7, characterized in that the alkoxylation is carried out at temperatures of 120 to 220°C and under pressures of 1 to 5 bar.

9. A process as claimed in at least one of claims 1 to 8, characterized in that the alkoxylation is carried out in the presence of 0.1 to 5.0% by weight, based on the end product of the alkoxylation reaction, of the alkaline earth metal phosphate.

10. A process as claimed in at least one of claims 1 to 9, characterized in that the alkaline earth metal phosphates are heated at temperatures of 180 to 300°C.

## Revendications

1. Procédé amélioré de préparation de composés tensioactifs non ioniques par alcoxylation de composés ayant des atomes d'hydrogène actifs en présence de sels de métal alcalino-terreux difficilement solubles dans le mélange réactionnel,
caractérisé en ce que
l'on utilise des phosphates de métal alcalino-terreux recuits de 180 à 300°C comme catalyseurs d'alcoxylation et que l'on sépare ceux-ci après achèvement de l'alcoxylation, par filtration.

2. Procédé selon la revendication 1,
caractérisé en ce que
les composés avec des atomes d'hydrogène actifs représentent des alcools primaires.

3. Procédé selon au moins une des revendications 1 et 2,
caractérisé en ce que
l'on effectue l'alcoxylation avec de l'oxyde d'éthylène et/ou de l'oxyde de propylène.

4. Procédé selon au moins une des revendications 1 à 3,
caractérisé en ce que
l'on utilise comme catalyseur d'alcoxylation du phosphate de strontium recuit.

5. Procédé selon au moins une des revendications 1 à 4,
caractérisé en ce que
les composés tensioactifs non ioniques suivent la formule (I) : 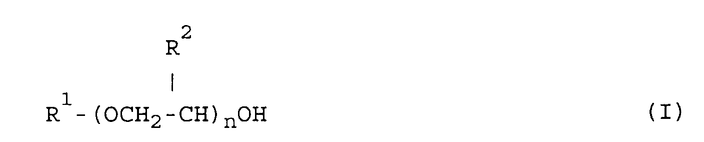 dans laquelle R¹ représente un reste hydrocarboné aliphatique ou cycloaliphatique, linéaire ou ramifié, ayant de 6 à 44 atomes de carbone et 0, 1, 2 ou 3 double liaisons, R² représente de l'hydrogène ou un radical méthyle et n représente des nombres entiers ou fractionnaires allant de 1 à 20.

6. Procédé selon au moins une des revendications 1 à 5,
caractérisé en ce que
R¹ représente un reste hydrocarboné, linéaire, saturé, aliphatique ayant de 12 à 14 atomes de carbone.

7. Procédé selon au moins une des revendications 1 à 5,
caractérisé en ce que
R¹ représente un reste hydrocarboné ramifié, saturé aliphatique ayant 16 à 36 atomes de carbone.

8. Procédé selon au moins une des revendications 1 à 7,
caractérisé en ce que
l'on effectue l'alcoxylation à des températures de 120 à 220°C et à des pressions de 1 à 5 bars.

9. Procédé selon au moins une des revendications 1 à 8,
caractérisé en ce que
l'on effectue l'alcoxylation en présence de 0,1 à 5,0 % en poids, rapporté au produit final de l'alcoxylation, de phosphate de métal alcalino-terreux.

10. Procédé selon au moins une des revendications 1 à 9,
caractérisé en ce que
l'on effectue le recuit des phosphates de métal alcalino-terreux à des températures de 180 à 300°C.
